(19) **Europäisches Patentamt**
**European Patent Office**
**Office européen des brevets**

(11) **EP 1 893 032 B1**

(12) # FASCICULE DE BREVET EUROPEEN

(45) Date de publication et mention de la délivrance du brevet:
**03.02.2010 Bulletin 2010/05**

(21) Numéro de dépôt: **06777244.2**

(22) Date de dépôt: **30.05.2006**

(51) Int Cl.:
*A23C 9/123* (2006.01)     *C07K 14/315* (2006.01)

(86) Numéro de dépôt international:
**PCT/EP2006/062715**

(87) Numéro de publication internationale:
**WO 2006/128864 (07.12.2006 Gazette 2006/49)**

(54) **SOUCHES MUTANTES DE BACTERIES LACTIQUES POSSEDANT UNE LACTOSE PERMEASE NON PHOSPHORYLABLE**

STAMMMUTANTEN VON MILCHSÄUREBAKTERIEN MIT NICHTPHOSPHORYLIERBARER LACTOSEPERMEASE

MUTANT STRAINS OF LACTIC ACID BACTERIA HAVING A NON-PHOSPORYLABLE LACTOSE PERMEASE

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **31.05.2005 FR 0505497**

(43) Date de publication de la demande:
**05.03.2008 Bulletin 2008/10**

(73) Titulaire: **COMPAGNIE GERVAIS DANONE**
**75009 Paris (FR)**

(72) Inventeurs:
• **DRUESNE, Anne**
**F-91440 Bures-sur-Yvette (FR)**
• **GARAULT, Peggy**
**F-91310 Montlhéry (FR)**
• **FAURIE, Jean-Michel**
**F-78350 Jouy-en-Josas (FR)**
• **LICAU, Nadine**
**91940 Les Ulis (FR)**

(74) Mandataire: **Vialle-Presles, Marie José et al**
**Cabinet ORES**
**36, rue de St Pétersbourg**
**75008 Paris (FR)**

(56) Documents cités:
**EP-A- 1 078 074     US-A- 5 639 648**

• **GUNNEWIJK M G ET AL: "Phosphorylation state of HPr determines the level of expression and the extent of phosphorylation of the lactose transport protein of Streptococcus thermophilus." THE JOURNAL OF BIOLOGICAL CHEMISTRY. 3 NOV 2000, vol. 275, no. 44, 3 novembre 2000 (2000-11-03), pages 34073-34079, XP002348522 ISSN: 0021-9258 cité dans la demande & POOLMAN B ET AL: "Regulation of bacterial sugar-H+ symport by phosphoenolpyruvate-depe ndent enzyme I/HPr-mediated phosphorylation." PROCEEDINGS OF THE NATIONAL ACADEMY OF SCIENCES OF THE UNITED STATES OF AMERICA. 31 JAN 1995, vol. 92, no. 3, 31 janvier 1995 (1995-01-31), pages 778-782, ISSN: 0027-8424**
• **POOLMAN B ET AL: "Lactose transport system of Streptococcus thermophilus. The role of histidine residues." THE JOURNAL OF BIOLOGICAL CHEMISTRY. 5 MAY 1992, vol. 267, no. 13, 5 mai 1992 (1992-05-05), pages 9150-9157, XP002348523 ISSN: 0021-9258**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

SEQUENCE LISTING

<110> COMPAGNIE GERVAIS DANONE
DRUESNE, Anne
GARAULT, Peggy
FAURIE, Jean-Michel
LICAU, Nadine

<120> SOUCHES MUTANTES DE BACTERIES LACTIQUES POSSEDANT UNE LACTOSE PERMEASE NON PHOSPHORYLABLE

<130> MJPmadSLP191/213

<150> FR0505497
<151> 2005-05-31

<160> 2

<170> Patents version 3.3

<210> 1
<211> 1220
<212> DNA
<213> Streptococcus thermophilus

<220>
<221> misc_feature
<222> (1)..(1220)

<223> Séquence du gène lacs de la souche mère I-2967

<400> 1

```
tccacaacct cttgtgttcc ttgttgtctt tatgattatc tctgactcag tagaatatgg      60
tcaatggaaa acgggacacc gtgatgaatc acttactttg tcagttcgtc cacttattga     120
taaacttggt ggtgcgatgt caaactggct tgtttctaca tttgccgtag ctgccggtat     180
gacaacaggt gcctcagcat caacaattac aacacatcaa cagtttatct ttaagcttgg     240
catgtttgct ttcccagcag caacaatgct tatcggtgcc ttcattgttg ctcgtaaaat     300
cactttgact gaagcacgtc acgctaaaat tgttgaagaa ttggaacatc gctttagcgt     360
agcaacttct gaaaatgaag ttaaagctaa cgtcgtatct cttgtaaccc ctacaactgg     420
ttatttggtt gatctctcaa gtgttaatga tgaacacttt gcttcaggta gcatgggtaa     480
aggtttcgcc attaaaccta ctgatggagc tgtctttgca ccaattagtg gtaccattcg     540
tcaaattctt cctactcgcc atgcagttgg tattgaaagt gaagatggtg tcattgttct     600
tatccacgtt ggcatcggaa cagttaaact taatggtgaa ggattcatta gttacgtaga     660
acaaggtgat catgttgaag ttggacaaaa acttcttgag ttctggtcac caattattga     720
gaaaaatggt cttgatgaca cagtacttgt cactgtaact aattcagaaa aattcagtgc     780
tttccatctt gaacaaaaag ttggagaaaa ggtagaagct ttgtctgaag ttattacctt     840
caaaaaagga gaataatcta tgaacatgac tgaaaaaatt caaacttatt taaacgatcc     900
aaagattgtt agcgttaata ctgttgatgc tcactcagat cataagtatt ttgaatctct     960
tgaagaattt tctgaagggg agatgaagtt aagacaatct cttaatggaa aatggaaaat    1020
tcactatgct cagaatacaa atcaggtttt aaaagacttt tataaaacag aatttgatga    1080
aactgatttg aatttcatca atgtaccagg tcatttagag cttcaaggtt ttggttctcc    1140
```

```
acaatatgtg aatacccaat atccttggga tggtaaagaa ttccttcgtc cacctcaagt    1200
tcctcaagaa tcaaatgctg                                                 1220
```

<210> 2
<211> 1278
<212> DNA
<213> Streptococcus thermophilus

<220>
<221> misc_feature
<222> (1)..(1278)
<223> séquence du gène lacs du variant I-3213

<400> 2

```
cttccaataa tcttgactgc agctgaactc ttcttcattc cacaacctct tgtgttcctt      60
gttgtcttta tgattatctc tgactcagta gaatatggtc aatggaaaac gggacaccgt     120
gatgaatcac ttactttgtc agttcgtcca cttattgata aacttggtgg tgcgatgtca     180
aactggcttg tttctacatt tgccgtagct gccggtatga caacaggtgc ctcagcatca     240
acaattacaa cacatcaaca gtttatcttt aagcttggca tgtttgcttt cccagcagca     300
acaatgctta tcggtgcctt cattgttgct cgtaaaatca ctttgactga agcacgtcac     360
gctaaaattg ttgaagaatt ggaacatcgc tttagcgtag caacttctga aaatgaagtt     420
aaagctaacg tcgtatctct tgtaacccct acaactggtt atttggttga tctctcaagt     480
gttaatgatg aacactttgc ttcaggtagc atgggtaaag gtttcgccat taaacctact     540
gatggagctg tctttgcacc aattagtggt accattcgtc aaattcttcc tactcgccat     600
gcagttggta ttgaaagtga agatggtgtc attgttctta tcgcggttgg catcggaaca     660
gttaaactta atggtgaagg attcattagt tacgtagaac aaggtgatca tgttgaagtt     720
ggacaaaaac ttcttgagtt ctggtcacca attattgaga aaaatggtct tgatgacaca     780
gtacttgtca ctgtaactaa ttcagaaaaa ttcagtgctt tccatcttga acaaaaagtt     840
ggagaaaagg tagaagcttt gtctgaagtt attaccttca aaaaaggaga ataatctatg     900
aacatgactg aaaaaattca aacttattta aacgatccaa agattgttag cgttaatact     960
gttgatgctc actcagatca taagtatttt gaatctcttg aagaattttc tgaaggggag    1020
atgaagttaa gacaatctct taatggaaaa tggaaaattc actatgctca gaatacaaat    1080
caggttttaa aagactttta taaaacagaa tttgatgaaa ctgatttgaa tttcatcaat    1140
gtaccaggtc atttagagct tcaaggtttt ggttctccac aatatgtgaa tacccaatat    1200
ccttgggatg gtaaagaatt ccttcgtcca cctcaagttc ctcaagaatc aaatgctgtt    1260
gcatcatacg ttaaacat                                                   1278
```

**Description**

La présente invention concerne le domaine de la fermentation laitière. Plus précisément, cette invention porte sur des nouveaux mutants de *Streptococcus thermophilus* exprimant une lactose perméase mutante, dont l'activité de transport du lactose est modifiée. Ces souches, et des ferments les comprenant, peuvent être utilisés pour l'obtention de produits laitiers fermentés possédant de bonnes propriétés de conservation.

Les yaourts (ou yoghourts) sont traditionnellement obtenus par fermentation du lait avec une association de différentes bactéries lactiques, choisies parmi les souches de *Streptococcus thermophilus* et de *Lactobacillus bulgaricus*. Au cours de la fermentation, qui est effectuée à une température d'environ 40 à 45°C, ces bactéries utilisent principalement le lactose comme substrat énergétique, et produisent de l'acide lactique qui entraîne la coagulation du lait ; lorsque le pH atteint une valeur d'environ 4,8 à 4,5, on met fin à cette étape de fermentation (également dénommée « acidification ») en refroidissant le produit. Celui-ci est ensuite maintenu au froid pendant la suite du processus de fabrication et de conditionnement, et jusqu'à sa consommation.

Cependant, le refroidissement ne stoppe pas complètement la fermentation lactique ; même lorsque le produit est maintenu à 4°C, on observe une augmentation progressive de son acidité au cours du temps.

Ce phénomène, connu sous le nom de post-acidification, est responsable d'une dégradation des qualités organoleptiques du produit pendant sa conservation.

La post-acidification résulte essentiellement de l'utilisation, par les bactéries, du lactose restant dans le produit à l'issue de l'étape d'acidification contrôlée. Pour l'éviter, il a été proposé d'utiliser des souches de bactéries lactiques ne fermentant pas, ou très peu, le lactose.

Les enzymes essentielles pour la fermentation du lactose chez *Streptococcus thermophilus* et *lactobacillus bulgaricus* sont codées par l'opéron lactose, qui contient le gène *lacS,* codant pour la lactose perméase, et le gène *lacZ,* codant pour la B-galactosidase. Ces protéines sont respectivement responsables du transport et de l'hydrolyse du lactose. Il a donc été proposé, pour obtenir des souches non-postacidifiantes de bactéries lactiques, de produire des variants artificiels, ou de sélectionner des mutants naturels, chez lesquels l'activité d'au moins une de ces enzymes était affectée.

Le Brevet EP 1078074 (Compagnie Gervais Danone) concerne des mutants de *L. bulgaricus* déficients en activité B-galactosidase, comprenant une mutation non-sens dans au moins un des gènes de l'opéron lactose. Ce brevet décrit plus spécifiquement un mutant dont l'analyse de la séquence fait apparaître deux mutations ponctuelles : l'une introduisant un codon de terminaison dans le gène de la B-galactosidase, qui induit l'incapacité de ce mutant à utiliser le lactose ; l'autre mutation induit un changement d'acide aminé au niveau du gène de la perméase (Lys -> Asn en position 122) ; EP 1078074 ne rapporte aucun effet de cette mutation sur le phénotype du mutant.

WO0188150 décrit des mutants d'une souche de *Lactobacillus.* Ces mutants sont incapables d'utiliser le lactose, mais conservent la capacité d'exprimer la β-galactosidase. WO0188150 ne précise pas la nature ou la position de la mutation concernée, et indique simplement qu'elle peut se situer dans un des gènes de structure de l'opéron lactose, par exemple la perméase, ou dans une région de régulation de l'opéron lactose, ou dans un gène intervenant dans le contrôle de l'expression de l'opéron lactose.

Les mutants décrits dans les documents cités ci-dessus ont en commun la propriété d'être complètement incapables d'utiliser le lactose. Ils ne peuvent pousser sur du lait que si celui-ci est complémenté avec un sucre autre que le lactose, généralement du glucose. Les propriétés d'acidification et de post-acidification de ces mutants sont contrôlées par la quantité de glucose ajoutée.

Afin de fournir une alternative à ces mutants, les Inventeurs ont recherché s'il était possible d'obtenir des souches de bactéries lactiques ayant d'une part une capacité à utiliser le lactose au cours de leur croissance comparable à celle des souches sauvages, et d'autre part une aptitude restreinte à utiliser le lactose au cours de la phase stationnaire, de façon à diminuer ou abolir le phénomène de post-acidification. Dans ce but, ils se sont intéressés à la possibilité d'agir sur la régulation du transport du lactose dans les cellules de bactéries lactiques, et notamment dans les cellules de S. *thermophilus.*

Le transport du lactose extracellulaire dans des cellules de S. *thermophilus* se fait par l'intermédiaire de la lactose perméase LacS. Ce transport du lactose se fait en symport avec un proton, ou en antiport avec le galactose intracellulaire résultant de la dégradation du lactose.

Le transport du lactose est dépendant de deux phénomènes : d'une part, l'état de phosphorylation de la lactose perméase et, d'autre part, l'expression du gène *lacS,* codant pour cette lactose perméase. Ces deux aspects sont détaillés ci-dessous.

*Phosphorylation de la lactose perméase*

La protéine LacS est composée d'un domaine de translocation et d'un domaine de régulation (IIA). Ces domaines contiennent différents résidus histidine, dont la phosphorylation intervient dans la régulation du transport du lactose. Notamment, le domaine IIA est phosphorylable sur l'histidine 552. Cette phosphorylation est effectuée par la protéine HPr *(histidine-containing phosphocarrier protein),* elle-même préalablement phosphorylée.

HPr peut être phosphorylée :

- sur une sérine par une protéine kinase ATP dépendante ; la réaction inverse d'hydrolyse de HPr(Ser-P) est catalysée par une activité phosphatase (HPr(Ser-P) phosphatase) ;
- sur une histidine HPr(His-P), par un groupement phosphoryle provenant du phosphoénolpyruvate, et par l'intermédiaire de l'enzyme I (EI).

Seule la forme de HPr phosphorylée sur l'histidine permet la phosphorylation de la lactose perméase sur l'histidine 552.

Il a été observé, sur un modèle *in vitro* de protéoliposomes reconstituant l'environnement membranaire de la protéine LacS et sa phosphorylation par HPr(His~P) que cette phosphorylation n'à pas d'effet sur le transport du lactose en symport avec un proton (Gunnewijk and Poolman, 2000a), mais augmente d'un facteur 2 environ, le flux de l'échange lactose/galactose.

*Transcription du gène lacS*

La transcription de l'opéron lactose est induite par la croissance en milieu lactosé. Le promoteur des gènes *lacS* et Z contient un site *cre (catabolite responsive element)* qui permet une régulation par CcpA: CcpA réprime l'expression de *lacS* et de *lacZ*. CcpA a au contraire un effet activateur sur la transcription du gène codant pour la lactate déshydrogénase (van den Bogaard *et al.,* 2000).

La forme HPr(Ser-P) est capable d'interagir avec CcpA. Ces protéines ensemble vont permettre de former un complexe avec le site *cre,* ce qui entraîne une répression de la transcription du gène *lacS* (Jones *et al.,* 1997).

Il a été observé (Gunnewijk and Poolman, 2000b) que la forme HPr(Ser-P) est dominante au début de la phase exponentielle de croissance des cultures de S. *thermophilus* et diminue au cours de celle-ci, alors que la forme HPr (His-P) apparaît au cours de la phase exponentielle et est maximale à l'entrée en phase stationnaire. Le passage de HPr(Ser-P) à HPr(His~P) se fait en parallèle de la diminution du lactose et de l'augmentation du galactose dans le milieu de culture, et d'une très forte augmentation de l'expression de la lactose perméase.

Ainsi, l'état de phosphorylation de la protéine HPr apparaît jouer un rôle dans la régulation du transport du lactose, en compensant la diminution du lactose dans le milieu, à travers d'une part le niveau d'expression de la protéine LacS, et d'autre part la régulation de son activité.

Sur la base des observations rapportées ci-dessus, Gunnewijk et Poolman ont proposé le modèle suivant : lorsque le lactose est abondant dans le milieu, l'expression du gène *lacS* est réprimée par le complexe HPr(Ser-P)/ CcpA. Au cours de la fermentation, l'accumulation de galactose dans le milieu et la diminution du lactose disponible provoquent une diminution de la capacité de la bactérie à faire pénétrer le lactose (et donc une diminution de l'acidification du milieu). Cette diminution entraîne une baisse de l'activité glycolytique, et une chute de la concentration en ATP, ainsi qu'une hausse de la concentration en phosphate inorganique, entraînant une baisse de l'activité de HPr(Ser-P) kinase au profit de l'activité de HPr(Ser-P) phosphatase, ce qui aurait pour effet la diminution de la concentration en HPr(Ser-P). Cette diminution de la concentration en HPr(Ser-P) permet de lever la répression catabolique du gène *lacS* et par conséquent d'augmenter la production de lactose perméase. Parallèlement l'augmentation de HPr(His~P) permettrait d'augmenter la phosphorylation de la lactose perméase sur l'histidine 552, et donc la capacité de transport du lactose par antiport avec le galactose.

Ce modèle, suggérant que la phosphorylation de la lactose perméase sur histidine 552 par HPr(His-P) augmente le flux du lactose dans les cellules lorsque la quantité de substrat dans le milieu diminue, permet de supposer que l'acidification en fin de phase exponentielle pourrait être ralentie si l'on empêche cette phosphorylation. Toutefois, il est basé en partie sur des expérimentations *in vitro,* et ne permet pas de préjuger de la part réelle *in vivo* de l'augmentation de la phosphorylation de la lactose perméase, par rapport à l'augmentation de son expression, dans l'importation du lactose *in vivo.* En outre, les observations concernant les effets de la concentration en HPr(Ser-P) et HPr(His-P) sur l'augmentation de l'expression et de la phosphorylation de LacS ont été effectuées sur des bactéries en phase exponentielle ou en début de phase stationnaire ; aucune indication n'est fournie en ce qui concerne les concentrations de ces deux formes de HPr à des stades plus avancés de la phase stationnaire.

Les seules informations disponibles quant à l'effet de l'absence de phosphorylation de LacS sur l'histidine 552 sont fournies dans une publication de Poolman, et al. (Poolman *et al.,* 1992), qui décrit différents plasmides contenant la séquence codant pour l'enzyme LacS de *Streptococcus thermophilus,* mutée sur différents résidus histidine. Ces plasmides sont utilisés pour transformer une souche *d'E.coli* dans laquelle le gène *lacS* endogène a été préalablement délété. Le transport du lactose dans les souches transformées par les différents mutants est évalué par rapport à celui observé dans la même souche *d'E.coli* contenant un plasmide codant pour l'enzyme LacS sauvage de *Streptococcus thermophilus.* Aucune différence significative n'est observée en ce qui concerne le mutant H552R dans lequel l'histidine native est remplacée par une arginine. Poolman et al. attribuent ce résultat soit à l'inefficacité de la phosphorylation de l'enzyme LacS sauvage de *Streptococcus thermophilus* par HPr(His-P) de *E.coli,* soit au fait que cette phosphorylation ne joue pas de rôle dans le transport du lactose.

Les Inventeurs ont cependant recherché si une mutation prévenant la phosphorylation de LacS sur le résidu histidine, pourrait avoir un effet sur les propriétés d'acidification et de post-acidification de la bactérie mutante.

Ils ont choisi pour cette étude une souche industrielle de *Streptococcus thermophilus.* Cette souche, déposée à la CNCM le 12/12/2002, sous le numéro I-2967, permet d'obtenir des produits laitiers fermentés ayant une texture intéressante ; toutefois, cette souche conduit à une post-acidification importante.

Les inventeurs ont construit et caractérisé un mutant de cette souche, exprimant, à la place de la lactose perméase sauvage, une lactose perméase mutée, non-phosphorylable sur l'histidine 552.

Ils ont constaté que cette souche mutante possédait une courbe d'acidification différente de celle de la souche mère. L'acidification démarre plus lentement dans le cas du mutant que dans celui de la souche mère, et la vitesse maximale d'acidification du mutant est moins élevée. Cependant, un pH équivalent est atteint au bout de 6 heures de fermentation pour les deux souches. C'est au niveau de la post-acidification que la différence entre les deux souches est la plus nette. Dans les mêmes conditions de conservation (28 jours de conservation à 10°C), le ΔpH (différence entre le pH à J0 et le pH à J28) est de l'ordre de 0,6 dans le cas de la souche mère, et de l'ordre de 0,4 dans le cas de la souche mutante. Cette différence de post-acidification ne provient pas d'une différence au niveau de la survie des bactéries. Celle-ci est en effet équivalente pour les deux souches. En outre, les produits fermentés obtenus avec le mutant présentent les mêmes qualités de texture que ceux obtenus avec la souche mère.

La présente invention porte donc en premier lieu sur un procédé d'obtention d'une souche mutante de bactérie lactique possédant une post-acidification plus faible que la souche mère dont elle est issue, caractérisé en ce que l'on introduit dans l'ADN génomique, notamment l'ADN chromosomique, de ladite souche mère, une mutation du codon codant pour l'histidine phosphorylable par HPr(His~P) du domaine IIA de la lactose perméase, ladite mutation induisant le remplacement de ladite histidine par un acide aminé non phosphorylable.

Selon un mode de réalisation préféré de la présente invention, ladite souche est une souche de *Streptococcus thermophilus,* et ladite mutation induit le remplacement de l'histidine en position 552 de la lactose perméase par un acide aminé non phosphorylable.

Ledit acide aminé non-phosphorylable peut être n'importe quel acide aminé, à l'exception de la sérine, de la tyrosine, de l'histidine et de la thréonine. De préférence, on choisira l'alanine.

Avantageusement, le codon codant pour l'histidine en position 552 de la lactose perméase est remplacé par un codon codant pour une alanine. Cette mutation génère un site de restriction *Bst*UI qui facilite le criblage des mutants obtenus.

Le procédé conforme à l'invention peut être mis en oeuvre en utilisant des techniques conventionnelles de mutagénèse dirigée, notamment de mutagénèse par PCR, bien connues de l'homme du métier.

L'ADN muté ainsi obtenu est ensuite inséré dans un vecteur permettant l'intégration du gène dans le chromosome bactérien. Cette intégration s'effectue de préférence par recombinaison de l'insert porté par le vecteur avec la région homologue du chromosome bactérien.

Classiquement, on insère l'ADN muté dans un vecteur intégratif portant un marqueur de sélection (par exemple un gène de résistance à un antibiotique), et l'on introduit ce vecteur dans les bactéries chez lesquelles on souhaite effectuer la mutation. Celles-ci sont ensuite cultivées sur milieu sélectif (par exemple, si le marqueur de sélection est un gène de résistance à un antibiotique, en présence de l'antibiotique correspondant), et l'on récupère les bactéries qui sont capables de pousser dans ces conditions, qui sont celles qui ont intégré le vecteur par recombinaison homologue entre l'insert et la région homologue du chromosome bactérien. La structure intégrée dans le chromosome est constituée des séquences du vecteur flanquées d'une part par la séquence mutée provenant de l'insert, et d'autre part par la séquence homologue de la bactérie hôte.

Les bactéries ainsi sélectionnées sont cultivées sur milieu non-sélectif, afin de permettre l'excision des séquences provenant du vecteur, qui s'effectue par recombinaison homologue entre les régions flanquant ces séquences. La moitié des bactéries chez lesquelles cette recombinaison a eu lieu contient la séquence « sauvage » provenant de la bactérie hôte, et l'autre moitié contient la séquence mutée provenant de l'insert. Les bactéries portant la mutation sont ensuite sélectionnées par tout moyen approprié. Par exemple, si la mutation crée un site de restriction, la sélection peut s'effectuer sur la base de la présence de ce site de restriction dans un produit d'amplification par PCR de la région mutante.

Des vecteurs intégratifs sont disponibles pour de nombreuses bactéries lactiques. Classiquement, pour une espèce bactérienne donnée, un vecteur intégratif est un vecteur qui peut être introduit dans les bactéries de cette espèce, mais qui est incapable de s'y répliquer.

A titre d'exemples de vecteurs utilisables comme vecteurs intégratifs chez *Streptococcus thermophilus* on citera Pgem5, Puc19 (Mollet *et al.,* 1993), Pnd324 (Duan *et al.,* 1999).

Avantageusement, pour augmenter l'efficacité de transformation, on peut utiliser comme vecteur intégratif un vecteur à réplication conditionnelle chez la bactérie choisie. Dans ce cas, les bactéries dans lesquelles a été introduit le vecteur sont dans un premier temps cultivées dans des conditions permissives pour sa réplication, ce qui lui permet de s'établir dans ces bactéries transformées ; dans un deuxième temps les bactéries sont cultivées en conditions non-permissives pour la réplication du vecteur, et l'on peut, comme dans le cas des vecteurs intégratifs classiques effectuer la sélection des bactéries dans lesquelles le vecteur a été intégré dans le chromosome.

A titre d'exemples de vecteurs à réplication conditionnelle utilisables comme vecteurs intégratifs chez un grand nombre de bactéries lactiques, on citera les vecteurs thermosensibles décrits par BISWAS et al. et MAGUIN et al (Biswas *et al.,* 1993; Maguin *et al.,* 1996), ainsi que dans la Demande PCT WO 93/18164, ou les vecteurs pwv01 (Law *et al.,* 1995) et Puc122 (Frere *et al.,* 1998).

L'invention a également pour objet une souche de bactérie lactique susceptible d'être obtenue par un procédé conforme à l'invention.

Cette souche est caractérisée en ce qu'elle contient dans son ADN chromosomique, une mutation du codon codant pour l'histidine phosphorylable par HPr(His~~P) du domaine IIA de la lactose perméase, ladite mutation induisant le remplacement de ladite histidine par un acide aminé non phosphorylable.

Selon un mode de réalisation préféré de la présente invention, ladite souche est une souche de *Streptococcus thermophilus,* dans laquelle le gène de la lactose perméase contient une mutation induisant le remplacement de l'histidine en position 552 de la protéine par un acide aminé non phosphorylable.

Une souche de bactéries lactiques conforme à l'invention a été déposée selon le Traité de Budapest, le 10 mai 2004, auprès de la CNCM (Collection Nationale de Cultures de Microorganismes), 25 rue du Docteur Roux, à Paris, sous le numéro I-3213. Il s'agit d'une souche mutante de S. *thermophilus,* dérivée de la souche CNCM I-2967 (déposée à la CNCM le 12 décembre 2002), par l'introduction, par mutagénèse dirigée, d'une mutation remplaçant le codon histidine 552 par un codon alanine.

Les souches de bactéries lactiques conformes à l'invention ont au cours de leur phase de croissance, une activité lactose perméase similaire à celle de la souche mère dont elles sont issues. Elles possèdent donc des capacités d'assimilation du lactose et d'acidification, comparables à celle de la souche mère dont elles sont issues. En revanche, leur activité lactose perméase pendant la phase stationnaire est réduite par rapport à celle de la souche mère, ce qui entraîne une post-acidification réduite.

Avantageusement, les souches de bactéries lactiques conformes à l'invention sont issues de bactéries lactiques possédant une activité β-galactosidase, et conservent cette activité. Elles peuvent croître normalement sur du lait non supplémenté avec un sucre autre que le lactose.

De préférence, les souches de bactéries selon l'invention sont des mutants appropriés à l'industrie alimentaire (ou mutants *« food-grade »*). Ils sont avantageusement dérivés de souches bactériennes caractérisées, présentant des propriétés avantageuses de fermentation des produits laitiers.

La présente invention concerne également un ferment lactique comprenant au moins une souche de bactéries telle que décrite ci-dessus. Selon un mode de réalisation particulier, un ferment lactique selon l'invention comprend au moins une souche mutante de S. *thermophilus* exprimant une lactose perméase dont l'histidine 552 a été remplacée par un résidu non phosphorylable, associée à au moins une autre souche de bactérie lactique, par exemple une souche de *L. bulgaricus,* pouvant éventuellement posséder également une post acidification réduite (par exemple résultant de la mutation, conformément à l'invention, de la lactose perméase, ou bien résultant d'une mutation inactivant la p-galactosidase).

Un procédé de préparation d'un produit laitier fermenté, comprenant une étape au cours de laquelle on fermente du lait à l'aide d'un ferment lactique tel que décrit ci-dessus, fait également partie intégrante de la présente invention, ainsi que tout produit laitier fermenté susceptible d'être obtenu par un tel procédé, tel qu'un yaourt, un lait fermenté, une boisson fermentée, un kéfir, un fromage ou un lait infantile fermenté.

Les exemples expérimentaux qui suivent, illustrés par les figures, exposent plus en détail certains aspects de la présente invention, sans toutefois en limiter l'objet.

## Légende des figures

Figure 1 : Comparaison de la séquence du gène *lacS* du variant I-3213 avec la séquence du gène *lacS* de la souche mère I-2967.

Figure 2 : Comparaison des courbes d'acidification obtenues avec le mutant I-3213 et la souche mère I-2967.

Figure 3 : Comparaison des vitesses d'acidification du mutant I-3213 et de la souche mère I-2967.

Figure 4 : Suivi de l'acidité Dolic au cours de la conservation des produits. Comparaison du mutant I-3213 et de la souche mère I-2967.

Figure 5 : Principe de la mesure de la viscosité η d'un produit.

## EXEMPLES

### EXEMPLE 1 : FABRICATION DU MUTANT

La souche de départ est la souche de *S. thermophilus* I-2967, déposée à la CNCM le 12 décembre 2002.

Dans la séquence du gène *lacS,* le codon pour l'histidine 552 (l'histidine qui est phosphorylable) a été remplacé par un codon alanine, par double recombinaison. De plus, la mutation effectuée (remplacement du deuxième nucléotide du codon 522 par une cytosine, au lieu d'une adénine) a créé dans le gène un site de restriction *Bst*U1. Ceci a permis de sélectionner les clones ayant intégré la mutation désirée dans le gène *lacS.*

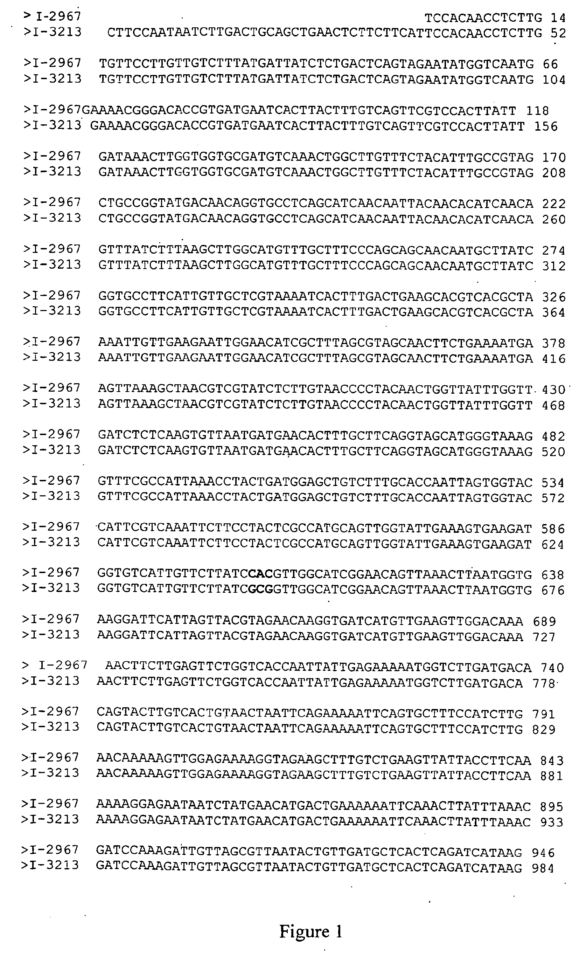

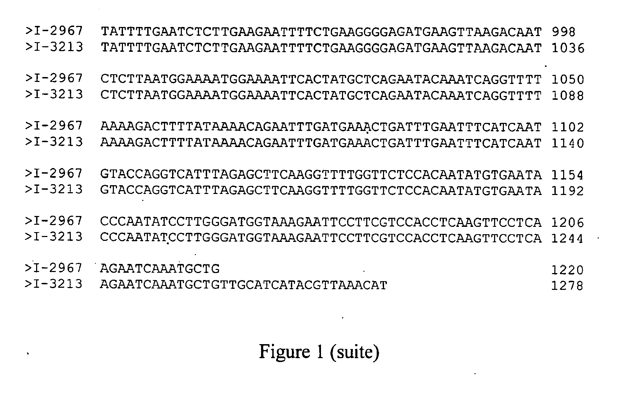

La stabilité de la mutation a été vérifiée à partir d'un des clones obtenus (déposé à la CNCM le 10 mai 2004 sous le numéro I-3213), par 6 repiquages successifs, suivis du séquençage du gène *LacS.* La figure 1 montre la comparaison de la séquence du gène *lacS* du variant I-3213 (SEQ ID NO : 2) avec celle du gène *lacS* de la souche mère I-2967 (SEQ ID NO : 1). La mutation est bien présente : le codon pour l'histidine 552 code désormais pour une alanine. Il n'y a pas de mutation indésirable ailleurs dans le gène *lacS.*

Le mutant I-3213 est approprié à l'utilisation agroalimentaire, car il ne contient aucune séquence résiduelle du plasmide utilisé pour intégrer la mutation du gène *lacS.*

### EXEMPLE 2 : TESTS PHYSIOLOGIQUES REALISES SUR LE MUTANT I-3213

Afin de vérifier que le mutant I-3213 est moins post-acidifiant que la souche mère I-2967, des produits sont fabriqués en souche pure et sont suivis jusqu'à J+28 : acidification, post-acidification, survie, texture.

#### 2.A - Protocole

- Revivification des souches par 2 repiquages.
- Préparation de ferments sur lait stérile additionné d'extraits de levure avec incubation à 44°C, jusqu'à ce qu'une acidité de 85°D soit atteinte (correspondant à $10^8$ à $10^9$ UFC/ml).
- Ensemencement d'un mélange de 120g de poudre de lait écrémé + 930 ml d'eau + 1g peptone de gélatine N3 (Organotechnie), pasteurisé 10 min à 95°C, avec 1% (v/v) de ferment.
- Incubation en pots dans une étuve à 44°C, jusqu'à ce qu'un pH de 4,65 soit atteint.
- Arrêt de la fermentation en plaçant les pots pendant 30 minutes dans l'eau glacée.
- Conservation des produits pendant 28 jours en chambre froide à 10°C.

#### 2.B - Suivi - Comparaison de l'acidification par les mutants par rapport à la souche I-2967

##### *2.B.1. Courbes d'acidification*

Grâce au système CINAC (CINétique d'ACidification, Alliance Instruments), le pH est mesuré en continu au cours du temps. On peut obtenir ainsi :

- la courbe d'acidification de chaque souche,
- la dérivée première par rapport au temps, qui donne la vitesse d'acidification.

##### *2.B.2. Mesure du pH*

Le suivi de l'évolution du pH au cours de la conservation des produits se fait grâce à un pH-mètre MP220 de chez Mettler Toledo.

##### *2.B.3. Mesure de l'acidité Dornic*

La mesure de l'acidité Dornic (D°) permet de titrer la concentration molaire en ions $H_3O^+$. Le nombre de degrés Dornic correspond au nombre, en dixièmes de millilitres, de solution d'hydroxyde de sodium, à la concentration 0,1 N qui sont nécessaires pour neutraliser 10,32g de lait. La neutralité est visualisée en utilisant un indicateur coloré, la phénolphtaléine. Aux alentours de sa zone de virage (pH 8,2), la phénolphtaléine passe de l'incolore au rose. Un degré Dornic représente 100 mg d'acide lactique par litre de lait.

### 2.B.4. Préparation du mix pour les tests produits

Le milieu lait est reconstitué à partir de 120 g de poudre de lait écrémé (Milex 240, Arla Food Ingredients) + 930 ml d'eau permutée + 1 g de peptide N3 (Vital Armor 950, Armor Protéines). Le mix est mélangé jusqu'à complète homogénéisation. Le milieu est ensuite réhydraté 30 min à température ambiante, puis pasteurisé à 95°C pendant 10 minutes.

### 2.B.5. Survie des souches

Les mesures de survie sont réalisées sur milieu gélosé M17 saccharosé. Isolement en surface via un ensemenceur spirale (WASP de chez AES). Incubation à 37°C sous H2CO2. Lecture après 72 heures d'incubation.
Les courbes d'acidification en souches pures ont été réalisées en duplicate.

### 2.B.6. Interprétation

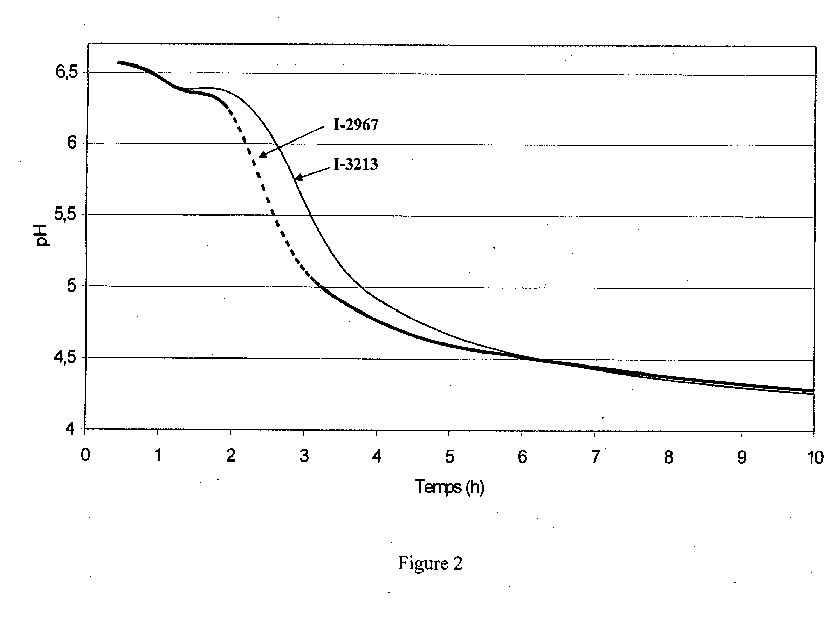

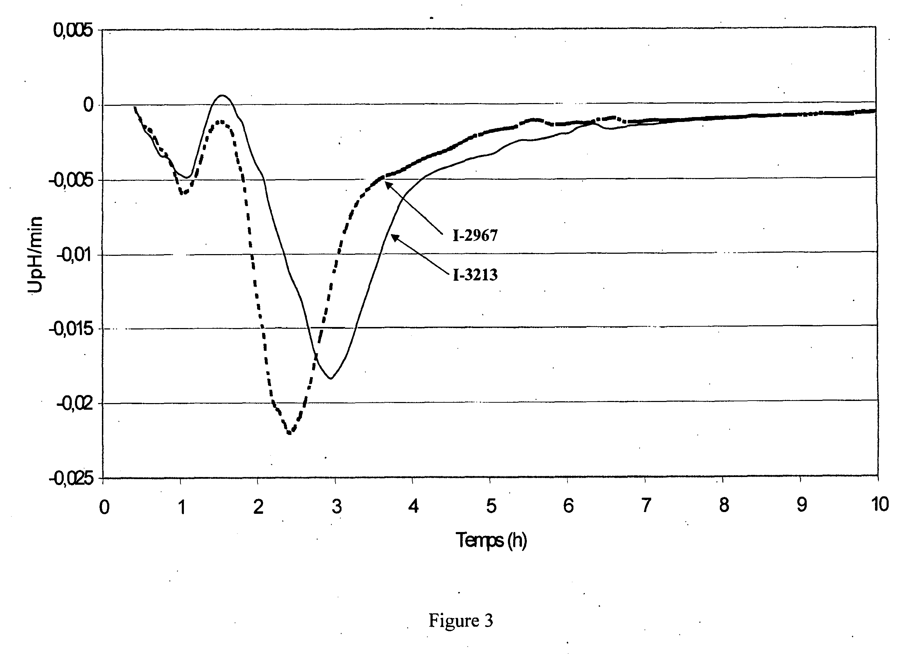

Les résultats, présentés aux figures 2 et 3, montrent que le mutant I-3213 a une courbe d'acidification plus lente que la souche mère, avec un effet urée plus prononcé et une vitesse maximale d'acidification plus faible. Cependant, le pH 4,50 est atteint en 6 heures pour les deux souches.

### 2.C - Suivi - Comparaison de la post-acidification du mutant I-3213 par rapport à la souche I-2967

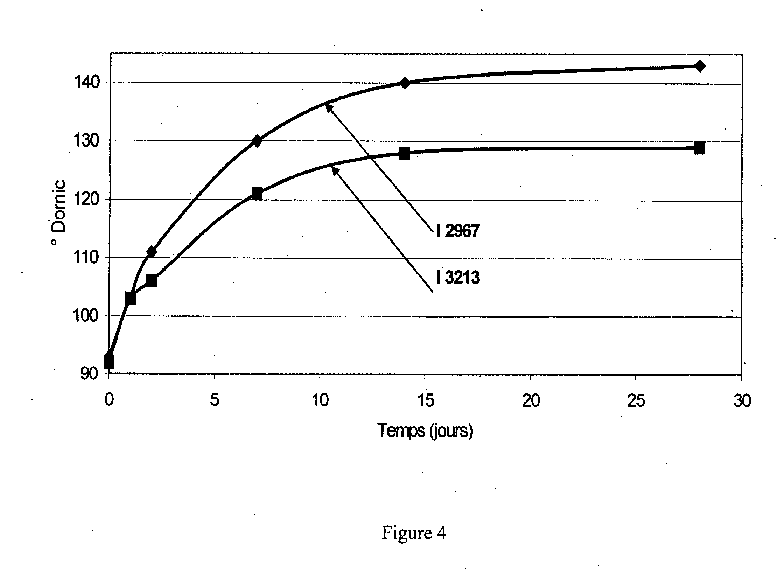

La figure 4 montre les résultats du suivi de l'acidité Dornic au cours de la conservation des produits, en comparant les produits obtenus par fermentation par le mutant I-3213 et par la souche mère I-2967.
Les résultats concernant la mesure du pH immédiatement après arrêt de la fermentation (J0), et après 28 jours de conservation à 10°C, sont présentés dans la Tableau 1 ci-dessous.

Tableau 1

|          | pH J0 | pH J28 |
|----------|-------|--------|
| I-2967 a | 4,65  | 4,07   |
| I-2967 b | 4,64  | 4,08   |
| I-3213 a | 4,60  | 4,27   |
| I-3213 b | 4,65  | 4,19   |

L'ensemble de ces résultats confirme que le mutant est moins post-acidifiant que la souche mère.

### 2.D - Suivi - Comparaison de la survie des mutants par rapport à la I-2967

Le tableau 2 ci-dessous indique le nombre de colonies bactériennes présentes dans 1 ml de produit après 28 jours de stockage à 10°C.

Tableau 2

|          | UFC/ml J28 |
|----------|------------|
| I-2967 a | $1,4.10^7$ |
| I-2967 b | $7,6.10^7$ |
| I-3213 a | $7,4.10^7$ |
| I-3213 b | $1,0.10^8$ |

Ces résultats montrent que la survie du mutant est aussi bonne que celle de la souche mère, après 28 jours de stockage suivant l'arrêt de la fermentation.

**2.E- Suivi - Comparaison de la texture des produits obtenus à partir d'un mutant en comparaison avec ceux obtenus à partir de la souche mère I-2967**

Les mesures de texture ont été réalisées sur les produits d'une seule fabrication. Toutes les mesures ont été effectuées en triple (3 pots par mesure).

Trois méthodes de mesure de la texture ont été réalisées sur les produits à J+7 :

- Mesure de pénétrométrie avec le TAXT2 (10°C)
- Mesure d'écoulement après brassage manuel sur le Rhéomat 260 (4°C)
- Mesure de viscosité sur le sérum après centrifugation sur le MCR300 (20°C)

Ces différentes techniques de mesure de la texture sont détaillées ci-dessous.

### 2.E.1- Mesure de viscosité sur le sérum des produits fermes

L'intérêt de cette méthode consiste à analyser le sérum des produits fermes afin de confirmer les caractérisations rhéologiques des laits fermentés par les souches.

Cette méthode d'analyse permet dans un premier temps de récupérer le sérum des produits fermes. Pour cela, une quantité de produit, environ 50g, est centrifugée à 631 g pendant 10 minutes à température ambiante, ce qui permet de recueillir le sérum présent à l'intérieur du gel des laits fermentés par les souches. Le sérum est alors prélevé et subit la même centrifugation afin d'éliminer la majorité des résidus du produit. Le reste de ces particules sédimente pour former un culot fragile.

La viscosité du sérum est ensuite mesurée à 20°C et à un gradient de cisaillement fixe de 100 $s^{-1}$ pendant une minute. Trois mesures sont également effectuées sur trois pots de lait fermenté par la même souche et dans les mêmes conditions. L'appareil utilisé pour cette analyse est un rhéomètre Anton Paar Physica® MCR 300 équipé d'une géométrie coaxiale à doubles entrefers de type DG 26.7/TEZ 150 p-c, ainsi que d'un système de régulation de température à effet Peltier. Ce système rotatif permet d'évaluer la viscosité du sérum à un gradient de cisaillement constant avec une acquisition d'un point par seconde.

Généralement, les deux premières valeurs de cette mesure sont incohérentes et faussées par l'initialisation du système rotatif. De ce fait, la viscosité de chaque sérum [Vs] est déterminée par la moyenne des valeurs retenues par l'appareil, excepté les deux premières.

### 2.E.2 - Mesure de pénétrométrie (Fgel - Dgel - F15mm)

L'appareil utilisé pour cette mesure est un pénétromètre Thermo Rhéo TAXT2 (Anton Paar Physica, Autriche).

Un cylindre d'environ 1 cm de diamètre pénètre dans le gel (tempéré à 10°C) à vitesse constante sur 15 mm de profondeur. Lors de la descente du mobile dans le produit, le gel oppose une résistance, il va se déformer avant de rompre. On mesure la force qui en résulte.

Les paramètres extraits sont les suivants :

Fgel = Force de gel (g), correspond à la valeur de la force appliquée par le mobile au moment de la rupture du gel (premier pic de la courbe).

Dgel = Distance à la force de gel (mm), correspond à la profondeur à laquelle se trouve le mobile au moment de la rupture du gel.

F15 = Force à 15 mm (g), correspond à la force mesurée lorsque le mobile est en fin de course.

### 2.E.3 - Mesure d'écoulement - Viscosité écoulement

Cette méthode consiste à déterminer la viscosité des produits fermes, après un brassage manuel et une incubation de 30 minutes à 4°C. Trois mesures sont effectuées à 4°C sur trois pots de lait fermenté par la même souche et dans les mêmes conditions. L'appareil utilisé pour cette analyse est un viscosimètre Mettler® RM 260 réfrigéré et équipé d'un système coaxial de type DIN 145. Ce système rotatif permet d'observer une destructuration du produit en fonction d'un gradient de cisaillement linéaire, soit une contrainte à un gradient donné.

Les résultats sont obtenus sous forme d'une courbe d'écoulement en continu, rampe montante et descendante entre 0 et 20 $s^{-1}$. Le produit subit un gradient de cisaillement croissant de 0 à 20 $s^{-1}$ pendant 1 minute. Cette phase correspond à la rampe montante. Puis, il subit un gradient de cisaillement décroissant de 20 à 0 $s^{-1}$ pendant 1 minute, correspondant à la rampe descendante.

Chaque courbe descendante est ensuite modélisée suivant le modèle de Casson :

$$\sqrt{\tau} = \sqrt{\tau_0} + \sqrt{\eta \times D}$$

$\tau$: Contrainte (Pa)

$\tau_0$ : Seuil d'écoulement du produit (Pa) [Seuil 4]

$\eta$ : Viscosité du produit (Pa.s) [V4]

D : Gradient de cisaillement (s-1)

Cette modélisation par Casson, suivie d'une droite de régression linéaire sur la partie descendante de la courbe, permet de relever un paramètre important qui est la viscosité du produit $\eta$, correspondant à la pente de la droite de régression.

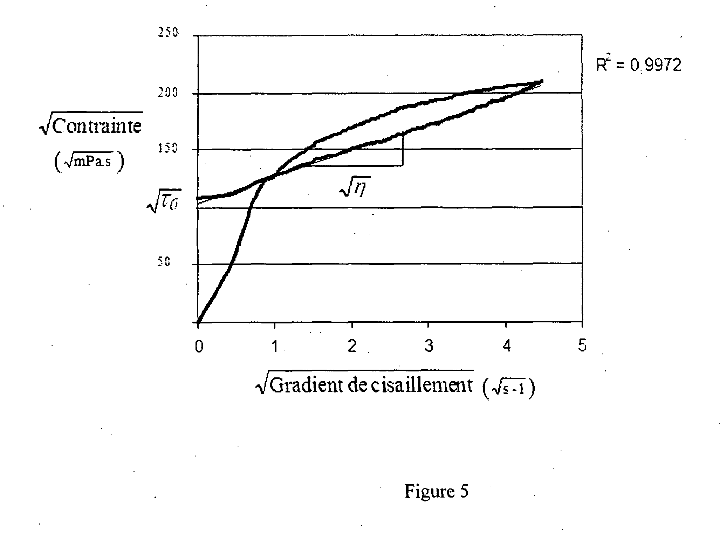

La figure 5 illustre le mode de calcul de la viscosité selon cette modélisation.

### *2.E.4 - Résultats*

Les résultats obtenus avec chacune des techniques de mesure sont présentés dans le Tableau 3 ci-après.

**Tableau 3**

| Suivis | I-2967 | I-3213 |
|---|---|---|
| Visco sérum (mPa.s) | 2,18 | 2,31 |
| Fgel (g) | 33,58 | 33,18 |
| Dgel (mm) | 3,29 | 2,96 |
| F 15m (g) | 39,23 | 38,78 |
| Viscosité écoulement | 1154 | 1171 |

Des analyses de variance (P<0,05) sont faites sur les résultats des mesures de texture (pour chaque paramètre les valeurs sont comparées par test de Student):

- Les paramètres Force de gel, Distance pour la force de gel et Force à 15mm montrent que les deux souches ne sont pas significativement différentes.
- Le paramètre de viscosité issu de la mesure d'écoulement montre que les deux souches ne sont pas significativement différentes.
- La viscosité du sérum étant très reproductible montre une différence significative entre les deux souches, mais le mutant ayant une viscosité plus importante que la souche I-2967, cela prouve qu'il n'y a pas eu de perte de texture.

### *2.E.5 - Interprétation*

Les mesures de texture effectuées sur les produits fermentés obtenus avec le mutant et la souche mère permettent de montrer qu'il n'y a pas de perte de texture due à la mutation.

### 2.F - Conclusions

Un mutant lactose perméase non phosphorylable de la souche I-2967 a été obtenu par double événement de recombinaison.

Ce mutant, nommé I-3213 présente :

- une courbe d'acidification différente de celle de la souche mère (vitesse ralentie),
- une post-acidification à J28 moins importante,
- une texture semblable à celle de la souche mère, et
- une bonne survie à J28.

### REFERENCES

# EP 1 893 032 B1

Biswas, I., Gruss, A., Ehrlich, S.D. and Maguin, E. (1993) High-efficiency gene inactivation and replacement system for gram-positive bacteria. J Bacteriol, 175, 3628-3635.

Duan, K., Liu, C.Q., Liu, Y.J., Ren, J. and Dunn, N.W. (1999) Nucleotide sequence and thermostability of pND324, a 3.6-kb plasmid from Lactococcus lactis. Appl Microbiol Biotechnol, 53, 36-42.

Frère, J., Benachour, A., Giard, J.C., Laplace, J.M., Flahaut, S. and Auffray, Y. (1998) A new theta-type thermosensitive replicon from Lactococcus lactis as an integration vector for Enterococcus faecalis. FEMS Microbiol Lett, 161, 107-114.

Gunnewijk, M.G. and Poolman, B. (2000a) HPr(His approximately P)-mediated phosphorylation differently affects counterflow and proton motive force-driven uptake via the lactose transport protein of Streptococcus thermophilus. J Biol Chem, 275, 34080-34085.

Gunnewijk, M.G. and Poolman, B. (2000b) Phosphorylation state of HPr détermines the level of expression and the extent of phosphorylation of the lactose transport protein of Streptococcus thermophilus. J Biol Chem, 275, 34073-34079.

Jones, B.E., Dossonnet, V., Kuster, E., Hillen, W., Deutscher, J. and Klevit, R.E. (1997) Binding of the catabolite repressor protein CcpA to its DNA target is regulated by phosphorylation of its corepressor HPr. J Biol Chem, 272, 26530-26535.

Law, J., Buist, G., Haandrikman, A., Kok, J., Venema; G. and Leenhouts, K. (1995) A system to generate chromosomal mutations in Lactococcus lactis which allows fast analysis of targeted genes. J Bacteriol, 177, 7011-7018.

Maguin, E., Prevost, H., Ehrlich, S.D. and Gruss, A. (1996) Efficient insertional mutagenesis in lactococci and other gram-positive bacteria. J Bacteriol, 178, 931-935.

Mollet, B., Knol, J., Poolman, B., Marciset, O. and Delley, M. (1993) Directed genomic integration, gene replacement, and integrative gene expression in Streptococcus thermophilus. J Bacteriol, 175, 4315-4324.

Poolman, B., Modderman, R. and Reizer, J. (1992) Lactose transport system of Streptococcus thermophilus. The role of histidine residues. J Biol Chem, 267, 9150-9157.

van den Bogaard, P.T., Kleerebezem, M., Kuipers, O.P. and de Vos, W.M. (2000) Control of lactose transport, beta-galactosidase activity, and glycolysis by CcpA in Streptococcus thermophilus: evidence for carbon catabolite repression by a non-phosphoenolpyruvate-dependent phosphotransferase system sugar. J Bacteriol, 182, 5982-5989.

**Revendications**

1.  Procédé d'obtention d'une souche mutante de bactérie lactique possédant une post-acidification plus faible que la souche mère dont elle est issue, **caractérisé en ce que** l'on introduit dans l'ADN génomique de ladite souche mère, une mutation du codon codant pour l'histidine phosphorylable par HPr(His~P) du domaine IIA de la lactose perméase, ladite mutation induisant le remplacement de ladite histidine par un acide aminé non phosphorylable.

2.  Procédé selon la revendication 1, **caractérisé en ce que** la souche mère est une souche de *Streptococcus thermophilus* et **en ce que** cette mutation introduit un codon alanine à la place du codon histidine 552.

3.  Souche de bactérie lactique susceptible d'être obtenue par un procédé selon l'une quelconque des revendications 1 ou 2.

4.  Souche de bactérie lactique selon la revendication 3, **caractérisée en ce qu'**elle possède une activité β-galactosidase.

5.  Souche de bactérie lactique selon la revendication 4, **caractérisée en ce qu'**il s'agit de la souche mutante de S. *thermophilus* déposée le 10 mai 2004 à la CNCM sous le numéro I-3213.

6. Ferment lactique comprenant au moins une souche de bactéries selon l'une quelconque des revendications 3 à 5.

7. Ferment lactique selon la revendication 6, **caractérisé en ce qu'**il comprend au moins une souche mutante de S. *thermophilus* selon l'une quelconque des revendications 3 à 5, associée à au moins une souche de *L. bulgaricus.*

8. Procédé de préparation d'un produit laitier fermenté, comprenant une étape au cours de laquelle on fermente du lait à l'aide d'un ferment lactique selon la revendication 6 ou la revendication 7.

9. Produit laitier fermenté susceptible d'être obtenu par le procédé selon la revendication 8.

10. Produit laitier fermenté selon la revendication 9, **caractérisé en ce que** ledit produit est choisi parmi les yaourts, les laits fermentés, les boissons fermentées, les kéfirs, les fromages et les laits infantiles fermentés.


**Claims**

1. Process for the preparation of a mutant strain of lactic acid bacteria having weaker post-acidification than the parent strain from which it is derived, **characterized in that** a mutation of the codon coding for the HPr(His-P)-phosphorylatable histidine of the IIA domain of lactose permease is introduced into the genomic DNA of said parent strain, said mutation inducing the replacement of said histidine by a non-phosphorylatable amino acid.

2. Process according to Claim 1, **characterized in that** the parent strain is a strain of *Streptococcus* thermophilus and **in that** this mutation introduces an alanine codon in place of histidine codon 552.

3. Strain of lactic acid bacteria obtainable by a process according to Claim 1 or 2.

4. Strain of lactic acid bacteria according to Claim 3, **characterized in that** it has β-galactosidase activity.

5. Strain of lactic acid bacteria according to Claim 4, **characterized in that** it is the mutant strain of *S. thermophilus* deposited in the CNCM on 10 May 2004 under the number I-3213.

6. Lactic ferment comprising at least one strain of bacteria according to any one of Claims 3 to 5.

7. Lactic ferment according to Claim 6, **characterized in that** it comprises at least one mutant strain of S. *thermophilus* according to any one of Claims 3 to 5, associated with at least one strain of *L. bulgaricus.*

8. Process for the preparation of a fermented dairy product, comprising a step during which milk is fermented using a lactic ferment according to Claim 6 or Claim 7.

9. Fermented dairy product obtainable by the process according to Claim 8.

10. Fermented dairy product according to Claim 9, **characterized in that** it is selected from yogurts, fermented milks, fermented drinks, kefirs, cheeses and fermented infant milks.


**Patentansprüche**

1. Verfahren zur Herstellung eines Mutantenstamms von Milchsäurebakterien, der eine schwächere Nachsäuerung besitzt als der Mutterstamm, von dem er stammt, **dadurch gekennzeichnet, dass** man in die genomische DNA des Mutterstamms eine Mutation des Codons, das für das Histidin, das durch HPr(His~P) phosphorylierbar ist, der Domäne ILA der Laktosepermease codiert, einführt, wobei die Mutation den Ersatz des genannten Histidins durch eine nicht phosphorylierbare Aminosäure herbeiführt.

2. Verfahren gemäß Anspruch 1, **dadurch gekennzeichnet, dass** der Mutterstamm ein Stamm von *Streptococcus* thermophilus ist und das dieser Mutation ein Alanin-Codon an die Stelle des Histidin-Codons 552 einführt.

3. Milchsäurebakterienstamm, der durch ein Verfahren gemäß einem der Ansprüche 1 oder 2 erhalten werden kann.

**4.** Milchsäurebakterienstamm gemäß Anspruch 3, **dadurch gekennzeichnet, dass** er β-Galactosidase-Aktivität besitzt.

**5.** Milchsäurebakterienstamm gemäß Anspruch 4, **dadurch gekennzeichnet, dass** es sich um den Mutantenstamm von *S. thermophilus* handelt, der am 10. Mai 2004 bei der CNCM unter der Nummer 1-3213 hinterlegt wurde.

**6.** Milchferment, umfassend wenigstens einen Bakterienstamm gemäß einem der Ansprüche 3 bis 5.

**7.** Milchferment gemäß Anspruch 6, **dadurch gekennzeichnet, dass** es wenigstens einen Mutantenstamm von S. *thermophilus* gemäß einem der Ansprüche 3 bis 5 in Kombination mit wenigstens einem L.-bulgaricus-Stamm umfasst.

**8.** Verfahren zur Herstellung eines fermentierten Milchprodukts, das eine Stufe umfasst, in deren Verlauf man Milch mit Hilfe eines Milchferments gemäß Anspruch 6 oder Anspruch 7 fermentiert.

**9.** Fermentierte Milchprodukt, das durch das Verfahren gemäß Anspruch 8 erhalten werden kann.

**10.** Fermentiertes Milchprodukt gemäß Anspruch 9, **dadurch gekennzeichnet, dass** das Produkt aus Joghurts, fermentierte-r Milch, fermentierten Getränken, Kefir, Käse und fermentierter Säuglingsmilch ausgewählt ist.

```
> I-2967                                                    TCCACAACCTCTTG 14
>I-3213     CTTCCAATAATCTTGACTGCAGCTGAACTCTTCTTCATTCCACAACCTCTTG 52

>I-2967     TGTTCCTTGTTGTCTTTATGATTATCTCTGACTCAGTAGAATATGGTCAATG 66
>I-3213     TGTTCCTTGTTGTCTTTATGATTATCTCTGACTCAGTAGAATATGGTCAATG 104

>I-2967GAAAACGGGACACCGTGATGAATCACTTACTTTGTCAGTTCGTCCACTTATT 118
>I-3213 GAAAACGGGACACCGTGATGAATCACTTACTTTGTCAGTTCGTCCACTTATT 156

>I-2967     GATAAACTTGGTGGTGCGATGTCAAACTGGCTTGTTTCTACATTTGCCGTAG 170
>I-3213     GATAAACTTGGTGGTGCGATGTCAAACTGGCTTGTTTCTACATTTGCCGTAG 208

>I-2967     CTGCCGGTATGACAACAGGTGCCTCAGCATCAACAATTACAACACATCAACA 222
>I-3213     CTGCCGGTATGACAACAGGTGCCTCAGCATCAACAATTACAACACATCAACA 260

>I-2967     GTTTATCTTTAAGCTTGGCATGTTTGCTTTCCCAGCAGCAACAATGCTTATC 274
>I-3213     GTTTATCTTTAAGCTTGGCATGTTTGCTTTCCCAGCAGCAACAATGCTTATC 312

>I-2967     GGTGCCTTCATTGTTGCTCGTAAAATCACTTTGACTGAAGCACGTCACGCTA 326
>I-3213     GGTGCCTTCATTGTTGCTCGTAAAATCACTTTGACTGAAGCACGTCACGCTA 364

>I-2967     AAATTGTTGAAGAATTGGAACATCGCTTTAGCGTAGCAACTTCTGAAAATGA 378
>I-3213     AAATTGTTGAAGAATTGGAACATCGCTTTAGCGTAGCAACTTCTGAAAATGA 416

>I-2967     AGTTAAAGCTAACGTCGTATCTCTTGTAACCCCTACAACTGGTTATTTGGTT 430
>I-3213     AGTTAAAGCTAACGTCGTATCTCTTGTAACCCCTACAACTGGTTATTTGGTT 468

>I-2967     GATCTCTCAAGTGTTAATGATGAACACTTTGCTTCAGGTAGCATGGGTAAAG 482
>I-3213     GATCTCTCAAGTGTTAATGATGAACACTTTGCTTCAGGTAGCATGGGTAAAG 520

>I-2967     GTTTCGCCATTAAACCTACTGATGGAGCTGTCTTTGCACCAATTAGTGGTAC 534
>I-3213     GTTTCGCCATTAAACCTACTGATGGAGCTGTCTTTGCACCAATTAGTGGTAC 572

>I-2967     CATTCGTCAAATTCTTCCTACTCGCCATGCAGTTGGTATTGAAAGTGAAGAT 586
>I-3213     CATTCGTCAAATTCTTCCTACTCGCCATGCAGTTGGTATTGAAAGTGAAGAT 624

>I-2967     GGTGTCATTGTTCTTATCCACGTTGGCATCGGAACAGTTAAACTTAATGGTG 638
>I-3213     GGTGTCATTGTTCTTATCGCGGTTGGCATCGGAACAGTTAAACTTAATGGTG 676

>I-2967     AAGGATTCATTAGTTACGTAGAACAAGGTGATCATGTTGAAGTTGGACAAA 689
>I-3213     AAGGATTCATTAGTTACGTAGAACAAGGTGATCATGTTGAAGTTGGACAAA 727

> I-2967    AACTTCTTGAGTTCTGGTCACCAATTATTGAGAAAAATGGTCTTGATGACA 740
>I-3213     AACTTCTTGAGTTCTGGTCACCAATTATTGAGAAAAATGGTCTTGATGACA 778

>I-2967     CAGTACTTGTCACTGTAACTAATTCAGAAAAATTCAGTGCTTTCCATCTTG 791
>I-3213     CAGTACTTGTCACTGTAACTAATTCAGAAAAATTCAGTGCTTTCCATCTTG 829

>I-2967     AACAAAAAGTTGGAGAAAAGGTAGAAGCTTTGTCTGAAGTTATTACCTTCAA 843
>I-3213     AACAAAAAGTTGGAGAAAAGGTAGAAGCTTTGTCTGAAGTTATTACCTTCAA 881

>I-2967     AAAAGGAGAATAATCTATGAACATGACTGAAAAAATTCAAACTTATTTAAAC 895
>I-3213     AAAAGGAGAATAATCTATGAACATGACTGAAAAAATTCAAACTTATTTAAAC 933

>I-2967     GATCCAAAGATTGTTAGCGTTAATACTGTTGATGCTCACTCAGATCATAAG 946
>I-3213     GATCCAAAGATTGTTAGCGTTAATACTGTTGATGCTCACTCAGATCATAAG 984
```

## Figure 1

```
>I-2967  TATTTTGAATCTCTTGAAGAATTTTCTGAAGGGGAGATGAAGTTAAGACAAT  998
>I-3213  TATTTTGAATCTCTTGAAGAATTTTCTGAAGGGGAGATGAAGTTAAGACAAT  1036

>I-2967  CTCTTAATGGAAAATGGAAAATTCACTATGCTCAGAATACAAATCAGGTTTT  1050
>I-3213  CTCTTAATGGAAAATGGAAAATTCACTATGCTCAGAATACAAATCAGGTTTT  1088

>I-2967  AAAAGACTTTTATAAAACAGAATTTGATGAAACTGATTTGAATTTCATCAAT  1102
>I-3213  AAAAGACTTTTATAAAACAGAATTTGATGAAACTGATTTGAATTTCATCAAT  1140

>I-2967  GTACCAGGTCATTTAGAGCTTCAAGGTTTTGGTTCTCCACAATATGTGAATA  1154
>I-3213  GTACCAGGTCATTTAGAGCTTCAAGGTTTTGGTTCTCCACAATATGTGAATA  1192

>I-2967  CCCAATATCCTTGGGATGGTAAAGAATTCCTTCGTCCACCTCAAGTTCCTCA  1206
>I-3213  CCCAATATCCTTGGGATGGTAAAGAATTCCTTCGTCCACCTCAAGTTCCTCA  1244

>I-2967  AGAATCAAATGCTG                                       1220
>I-3213  AGAATCAAATGCTGTTGCATCATACGTTAAACAT                   1278
```

Figure 1 (suite)

Figure 2

Figure 3

EP 1 893 032 B1

EP 1 893 032 B1

Figure 4

EP 1 893 032 B1

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 1078074 A
- WO 0188150 A
- WO 9318164 A
- FR 0505497

### Littérature non-brevet citée dans la description

- **Biswas, I. ; Gruss, A. ; Ehrlich, S.D. ; Maguin, E.** High-efficiency gene inactivation and replacement system for gram-positive bacteria. *J Bacteriol,* 1993, vol. 175, 3628-3635
- **Duan, K. ; Liu, C.Q. ; Liu, Y.J. ; Ren, J. ; Dunn, N.W.** Nucleotide sequence and thermostability of pND324, a 3.6-kb plasmid from Lactococcus lactis. *Appl Microbiol Biotechnol,* 1999, vol. 53, 36-42
- **Frère, J. ; Benachour, A. ; Giard, J.C. ; Laplace, J.M. ; Flahaut, S. ; Auffray, Y.** A new theta-type thermosensitive replicon from Lactococcus lactis as an integration vector for Enterococcus faecalis. *FEMS Microbiol Lett,* 1998, vol. 161, 107-114
- **Gunnewijk, M.G. ; Poolman, B.** HPr(His approximately P)-mediated phosphorylation differently affects counterflow and proton motive force-driven uptake via the lactose transport protein of Streptococcus thermophilus. *J Biol Chem,* 2000, vol. 275, 34080-34085
- **Gunnewijk, M.G. ; Poolman, B.** Phosphorylation state of HPr détermines the level of expression and the extent of phosphorylation of the lactose transport protein of Streptococcus thermophilus. *J Biol Chem,* 2000, vol. 275, 34073-34079
- **Jones, B.E. ; Dossonnet, V. ; Kuster, E. ; Hillen, W. ; Deutscher, J. ; Klevit, R.E.** Binding of the catabolite repressor protein CcpA to its DNA target is regulated by phosphorylation of its corepressor HPr. *J Biol Chem,* 1997, vol. 272, 26530-26535
- **Law, J. ; Buist, G. ; Haandrikman, A. ; Kok, J. ; Venema; G. ; Leenhouts, K.** A system to generate chromosomal mutations in Lactococcus lactis which allows fast analysis of targeted genes. *J Bacteriol,* 1995, vol. 177, 7011-7018
- **Maguin, E. ; Prevost, H. ; Ehrlich, S.D. ; Gruss, A.** Efficient insertional mutagenesis in lactococci and other gram-positive bacteria. *J Bacteriol,* 1996, vol. 178, 931-935
- **Mollet, B. ; Knol, J. ; Poolman, B. ; Marciset, O. ; Delley, M.** Directed genomic integration, gene replacement, and integrative gene expression in Streptococcus thermophilus. *J Bacteriol,* 1993, vol. 175, 4315-4324
- **Poolman, B. ; Modderman, R. ; Reizer, J.** Lactose transport system of Streptococcus thermophilus. The role of histidine residues. *J Biol Chem,* 1992, vol. 267, 9150-9157
- **van den Bogaard, P.T. ; Kleerebezem, M. ; Kuipers, O.P. ; de Vos, W.M.** Control of lactose transport, beta-galactosidase activity, and glycolysis by CcpA in Streptococcus thermophilus: evidence for carbon catabolite repression by a non-phosphoenolpyruvate-dependent phosphotransferase system sugar. *J Bacteriol,* 2000, vol. 182, 5982-5989